# EUROPÄISCHE PATENTSCHRIFT

(11) **EP 1 827 591 B1**
(45) Veröffentlichungstag und Bekanntmachung des Hinweises auf die Patenterteilung: **15.04.2015**
(21) Anmeldenummer: 05819414.3
(22) Anmeldetag: 09.12.2005
(51) Int. Cl.: A61N 1/08, A61N 1/36, A61N 1/04

(54) **VORRICHTUNG ZUR STEUERUNG DER ELEKTRISCHEN LADUNG AN STIMULATIONSELETRODEN**
DEVICE FOR CONTROLLING THE ELECTRIC CHARGE ON STIMULATION ELECTRODES
DISPOSITIF POUR REGULER LA CHARGE ELECTRIQUE AU NIVEAU D'ELECTRODES DE STIMULATION

(30) Priorität: 13.12.2004 DE 102004059973
(43) Veröffentlichungstag der Anmeldung: 05.09.2007
(73) Patentinhaber: Pixium Vision SA, 75012 Paris (FR)
(72) Erfinder: ROCKE, Andre, 30823 Garbsen (DE); ORTMANNS, Maurits, 31712 Niedernwöhren (DE); UNGER, Norbert, 38118 Braunschweig (DE)
(74) Vertreter: Graf von Stosch, Andreas
(86) Internationale Anmeldenummer: PCT/EP2005/013258
(87) Internationale Veröffentlichungsnummer: WO 2006/063743

(56) Entgegenhaltungen:
- US-A- 5 376 104
- US-A1- 2002 169 486
- US-B1- 6 473 649

## Beschreibung

Die vorliegende Erfindung betrifft eine Vorrichtung zur Steuerung der elektrischen Spannung bzw. der elektrischen Ladung an Stimulationselektroden, die der Stimulation von lebendem Gewebe oder Nerven dienen. Die vorliegende Erfindung betrifft insbesondere eine elektronische Schaltung zur Steuerung der elektrischen Ladung an Stimulationselektroden in einem System zur Stimulation von lebendem Gewebe oder Nerven durch einzelne oder wiederholte Stimulationsimpulse über Stimulationselektroden.

Es sind bereits Vorrichtungen in Form von Implantaten zur Stimulation von lebendem Gewebe bekannt. So wurden beispielsweise Implantate für die Netzhaut (Retina) des menschlichen Auges entwickelt, die zur Behandlung von Patienten vorgesehen sind, deren Sehvermögen teilweise oder vollständig durch Defekte in der Retina verloren gegangen ist. Im Prinzip wird dabei eine mikroelektronische Vorrichtung im Bereich der Retina mit einer Vielzahl von lichtempfindlichen Pixelelementen implantiert, über die ein auf die Retina durch die noch intakte Linse des Auges projiziertes Bild aufgenommen wird; alternativ kann die Bilderfassung auch durch eine externe Kamera erfolgen. Das durch die Pixelelemente bzw. die Kamera erfasste Bild wird in elektrische Signale umgesetzt und über Stimulationselektroden mittels elektrischer Stimulationsimpulse an das umgebende Gewebe bzw. die Zellen der Retina abgegeben, um so das Sehvermögen des erblindeten oder teilweise erblindeten Patienten wiederherzustellen bzw. zu verbessern.

Bei der Stimulation von lebendem Gewebe oder Nerven durch einzelne oder wiederholte Stimulationsimpulse über Stimulationselektroden können Unausgeglichenheiten elektrischer Ladungen an den Stimulationselektroden auftreten. Bei bekannten Stimulations-Systemen bzw. Stimulatoren werden häufig Impulsgeneratoren verwendet, um die elektrischen Stimulationsimpulse an den Stimulationselektroden zu erzeugen. Dabei wird die Form bzw. der Verlauf der elektrischen Stimulationsimpulse auf die Art des zu stimulierenden Gewebes angepasst. Über einen Stromgenerator werden die Stimulationselektroden mit elektrischem Strom beaufschlagt, der den vom Impulsgenerator erzeugten elektrischen Stimulationsimpulsen entspricht.

Nach einem einzelnen Stimulationsimpuls kann jedoch beispielsweise aufgrund von Fehlern oder Toleranzen eine geringe elektrische Ladung auf der Stimulationselektrode zurückbleiben. Eine kontinuierlich verbleibende oder zunehmende Unausgeglichenheit der elektrischen Ladungen an den Stimulationselektroden kann zu einem unerwünschtem Stromfluss zwischen den Stimulationselektroden und damit zu Schäden sowohl am Gewebe als auch an den Stimulationselektroden bis zur Zerstörung der Stimulationselektroden und zum totalen Ausfall des Stimulations-Systems führen. Aufgrund von Unausgeglichenheiten elektrischer Ladungen an den Stimulationselektroden kann es insbesondere zu einem Gleichstromfluss zwischen den Stimulationselektroden kommen, was bei dem Patienten mit unangenehmen Empfindungen und nachteiligen Wirkungen auf das Gewebe oder die Nerven verbunden sein kann.

Bei einigen bekannten Stimulations-Systemen erfolgt die Beseitigung von Unausgeglichenheiten elektrischer Ladungen an den Stimulationselektroden beispielsweise durch Elektrodenkurzschluss oder mittels Parallelwiderstände zum passiven Entladen der Stimulationselektroden oder von üblicherweise verwendeten Serienkapazitäten. Solche Vorrichtungen haben jedoch den Nachteil, dass sie mit einem hohen Platzbedarf verbunden sind, denn es ist grundsätzlich erstrebenswert, Stimulations-Systeme auf möglichst kleinem Raum unterzubringen.

US 6301505 B1 beschreibt eine Vorrichtung zur Stimulation von Nervengewebe, insbesondere im Innenohr oder von Muskelgewebe. Eine elektrische Schaltung überwacht den Potentialaufbau zwischen den Stimulationselektroden. Sobald ein zu hohes Potential zwischen den Stimulationselektroden erfasst wird, werden weitere Stimulationen verhindert, so dass sich die Potentialdifferenz zwischen den Stimulationselektroden nicht weiter aufbauen kann. Eine Stimulation der Stimulationselektroden wird dann so lange unterdrückt, bis sich die Potentialdifferenz durch einen Kurzschluss zwischen den Elektroden wieder ausgeglichen hat oder unterhalb des Grenzwerts liegt. Diese Vorrichtung hat den Nachteil, dass bis zum Ausgleich einer Potentialdifferenz zwischen den Stimulationselektroden keine Stimulation vorgenommen werden kann.

DE 10151650 A1 beschreibt eine Elektrodenanordnung zur elektrischen Stimulation mit einer Stimulationselektrode, über die biologischem Material ein Stimulussignal zugeführt wird, und einer Gegenelektrode. Zusätzlich ist die Elektrodenanordnung mit einer Sensorelektrode ausgestattet, mit der eine Polarisationsspannung an der Stimulationselektrode bestimmt wird, wodurch sich auch statische Anteile der Elektrodenpolarisation erfassen lassen. Nach diesem bekannten Verfahren wird das Polarisationspotential kontinuierliche gemessen und das Stimulationssignal derart beeinflusst, dass das Polarisationspotential zwischen den Stimulationselektroden einen bestimmten Wert nicht überschreitet. Dies wird entweder durch Justieren der Amplitude oder durch Abschalten des Stimulationssignals erreicht. Der Nachteil dieser Elektrodenanordnung besteht darin, dass mit der Sensorelektrode eine zusätzliche Elektrode zur Messung des Polarisationspotentials benötigt wird, was sowohl die Kosten der Stimulationsvorrichtung als auch den Aufwand bei der Implantation und die Beeinträchtigung des zu stimulierenden Gewebes erhöht. Ferner erfolgt bei dieser bekannten Methode die Messung einer Potentialdifferenz zwischen den Stimulationselektroden während der Stimulation, wodurch das Messergebnis beeinträchtigt werden kann.

Eine Vorrichtung gemäss der Präambel von Anspruch 1 ist bereits aus US6473649 bekannt.

Der vorliegenden Erfindung liegt die Aufgabe zugrunde, eine Vorrichtung zur Steuerung der elektrischen Ladung an Stimulationselektroden mit geringem Platzbedarf zu schaffen, die einen unerwünschten Stromfluss zwischen den Stimulationselektroden eines Stimulations-Systems aufgrund von Unausgeglichenheiten elektrischer Ladungen an den Stimulationselektroden reduziert bzw. beseitigt.

Diese Aufgabe wird durch die erfindungsgemäße Vorrichtung mit den Merkmalen gemäß Anspruch 1 sowie durch ein Verfahren mit den Merkmalen gemäß Anspruch 14 gelöst. Vorteilhafte Weiterbildungen der Erfindung sind jeweils in den Unteransprüchen angegeben.

Gemäß einem Aspekt der vorliegenden Erfindung wird die oben genannte Aufgabe durch eine Vorrichtung zur Stimulation von lebendem Gewebe oder Nerven durch einzelne oder wiederholte Stimulationsimpulse über Stimulationselektroden gelöst, die mit lebenden Nerven oder Gewebe kontaktiert sind, das durch die Stimulationsimpulse der Stimulationselektroden stimuliert wird, wobei die Vorrichtung eine elektrische Schaltung umfasst, welche die elektrische Spannung bzw. die elektrische Ladung an den Stimulationselektroden in Abhängigkeit von der elektrischen Spannung zwischen den Stimulationselektroden regelt und Unausgeglichenheiten elektrischer Ladungen an den Stimulationselektroden reduziert oder ausgleicht.

Gemäß einem weiteren Aspekt der vorliegenden Erfindung wird die oben genannte Aufgabe gelöst durch ein Verfahren zum Betreiben der oben genannten Vorrichtung umfassend die folgenden Schritte:
- Ermitteln einer elektrischen Spannung zwischen den Stimulationselektroden bzw. einer Unausgeglichenheit elektrischer Ladungen an den Stimulationselektroden,
- Vergleichen der ermittelten Spannung zwischen den Stimulationselektroden mit einem vorgegebenen Spannungsbereich,
- Erzeugen und Anlegen eines positiven oder negativen elektrischen Stroms bestimmter Dauer und Intensität an mindestens eine Stimulationselektrode, wodurch die elektrischen Spannung zwischen den Stimulationselektroden bzw. eine Unausgeglichenheit elektrischer Ladungen an den Stimulationselektroden reduziert oder ausgeglichen wird.

Nach der vorliegenden Erfindung wird folglich eine Vorrichtung sowie ein Verfahren zum Betreiben der Vorrichtung bereitgestellt, die in der Lage ist, die elektrische Ladung an Stimulationselektroden eines Systems zur Stimulation von lebendem Gewebe oder Nerven zu regeln und einen Ladungsausgleich an den Stimulationselektroden herbeizuführen. Die vorliegende Erfindung stellt insbesondere eine elektronische Schaltung bereit, die zur Steuerung und zum Ausgleich der elektrischen Ladung an Stimulationselektroden in einem System zur Stimulation von lebendem Gewebe oder Nerven durch einzelne oder wiederholte Stimulationsimpulse über Stimulationselektroden dient. Die erfindungsgemäße Vorrichtung ist damit in der Lage, einen Ausgleich der elektrischen Ladung an den Stimulationselektroden des Stimulations-Systems herbeizuführen.

Ein besonderer Vorteil der erfindungsgemäßen Vorrichtung besteht folglich darin, dass Unausgeglichenheiten elektrischer Ladungen an den Stimulationselektroden und die damit verbundenen nachteiligen Effekte auf das Gewebe und die Nerven vermieden werden, indem sie durch einen entsprechenden Ladungsausgleich aktiv beseitigt werden. Ein weiterer Vorteil der erfindungsgemäßen Vorrichtung besteht darin, dass sie keine Verwendung von Serienkapazitäten mehr erfordert und damit einen geringeren Platzbedarf hat als bekannte Vorrichtungen. Zwar ist die Verwendung von mindestens einem Parallelwiderstand nicht zwingend erforderlich, kann aber ggf. zur Erhöhung der Erstfehlersicherheit vorgesehen sein. Noch ein weiterer Vorteil der erfindungsgemäßen Vorrichtung besteht darin, dass neben den Stimulationselektroden keine zusätzliche Messelektrode benötigt wird.

Weitere Einzelheiten, bevorzugte Ausführungsformen und Vorteile der vorliegenden Erfindung ergeben sich aus der nachfolgenden Beschreibung unter Bezugnahme auf die Zeichnung. Es zeigen:
- Figur 1: ein schematisches Blockschaltbild einer elektrischen Schaltung für eine Vorrichtung gemäß einer bevorzugten Ausführungsform der vorliegenden Erfindung zur Verwendung in einem Stimulations-System;
- Figur 2: ein Elektrodenmodell in Form einer schematischen Darstellung mit einem elektrischen Ersatzschaltbild für eine Vorrichtung gemäß einer bevorzugten Ausführungsform der vorliegenden Erfindung in einem Stimulations-System; und
- Figur 3: eine elektrische Ersatzschaltung zur Darstellung unterschiedlicher Zustände der Stimulationselektroden eines Stimulations-Systems mit einer Vorrichtung gemäß einer bevorzugten Ausführungsform der vorliegenden Erfindung.

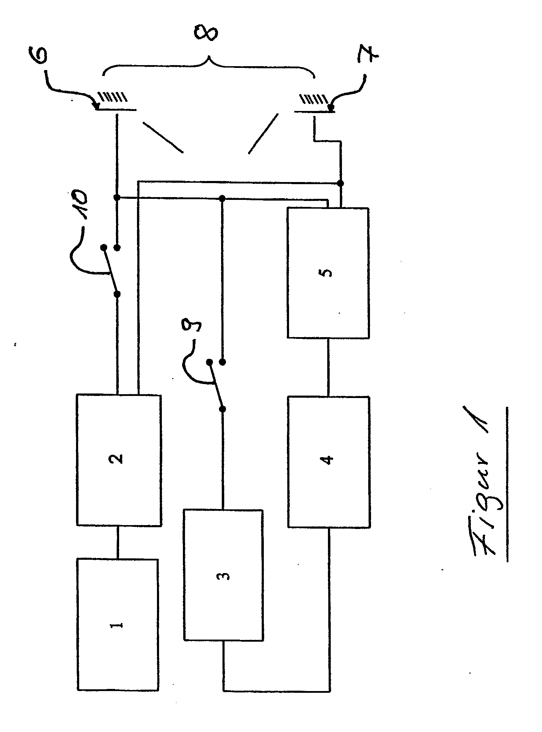

In Figur 1 ist ein schematisches Blockschaltbild einer elektrischen Schaltung für eine Vorrichtung gemäß einer ersten bevorzugten Ausführungsform der vorliegenden Erfindung dargestellt, das in einem Stimulations-System zur Stimulation von lebendem Gewebe oder Nerven durch einzelne oder wiederholte Stimulationsimpulse eingesetzt werden kann. Bei dieser bevorzugten Ausführungsform der vorliegenden Erfindung umfasst die erfindungsgemäße Vorrichtung einen Impulsgenerator 1, der elektrische Impulse erzeugt. Diese elektrischen Impulse werden vom Impulsgenerator 1 an eine Strom/Spannungsquelle 2 weitergeleitet, wo sie zu Stimulationsimpulsen verstärkt und durch elektrische Leitungen an eine erste Stimulationselektrode 6 und an eine zweite Stimulationselektrode bzw. Gegenelektrode 7 übertragen werden.

Die Stimulationselektroden 6, 7 sind beispielsweise mit menschlichen Nerven oder Gewebe 8 kontaktiert, das durch die Stimulationsimpulse der Stimulationselektroden 6, 7 stimuliert wird. Dabei ist die Form bzw. der Verlauf der vom Impulsgenerator 1 und der Strom/Spannungsquelle 2 erzeugten elektrischen Stimulationsimpulse auf die Art des zu stimulierenden Gewebes bzw. der zu stimulierenden Nerven angepasst. Die Verbindung über die elektrische Leitung zwischen der Strom/Spannungsquelle 2 und der Stimulationselektrode 6 kann durch einen Schaltkontakt 10 unterbrochen bzw. hergestellt werden.

Die in Figur 1 dargestellte Ausführungsform der erfindungsgemäßen Vorrichtung zur Stimulation von lebenden Gewebe oder Nerven umfasst ferner einen Ladungs- bzw. Spannungsmesser 5, der mit den beiden Stimulationselektroden 6 und 7 verbunden ist. Der Ladungs- bzw. Spannungsmesser 5 ermittelt die elektrische Spannung zwischen den Stimulationselektroden 6, 7 und ermittelt damit Unausgeglichenheiten elektrischer Ladungen bzw. Ladungsverschiedenheiten an den Stimulationselektroden 6, 7, die an einen Vergleicher 4 weitergeleitet werden. Der Vergleicher 4 stellt fest, ob die vom Ladungs- bzw. Spannungsmesser 5 ermittelte Spannung zwischen den Stimulationselektroden 6 und 7 unterhalb, innerhalb oder oberhalb eines vorgegebenen Spannungsbereichs liegt, der durch vorgegebene Grenzwerte definiert ist.

Der Vergleicher 4 ist mit einem Ladungsinjektor 3 verbunden, der einen elektrischen Strom bestimmter Dauer und Intensität erzeugen kann. Der Ladungsinjektor 3 ist wiederum über eine elektrische Leitung mit der Stimulationselektrode 6 verbunden, wobei die Verbindung zwischen dem Ladungsinjektor 3 und der Stimulationselektrode 6 durch einen Schaltkontakt 9 unterbrochen bzw. hergestellt werden kann. Aufgrund des vom Vergleicher 4 ermittelten Resultats bezüglich der Spannung zwischen den Stimulationselektroden 6 und 7 übermittelt der Vergleicher 4 dem Ladungsinjektor 3 ein entsprechendes Signal, woraufhin der Ladungsinjektor 3 einen positiven oder negativen elektrischen Strom bestimmter Dauer und Intensität an die Stimulationselektrode 6 anlegen kann.

Falls der Vergleicher 4 feststellt, dass die Spannung zwischen den Stimulationselektroden 6, 7 nicht innerhalb des vorgegebenen Spannungsbereichs liegt, legt der Ladungsinjektor 3 für eine bestimmte Zeitspanne einen entsprechenden elektrischen Strom bestimmter Intensität auf die Stimulationselektrode 6. Dabei wird die Ausgleichstromrichtung bzw. die Polarität des Ausgleichsstroms vom Ladungsinjektor 3 so gewählt, dass die absolute Spannung zwischen den Stimulationselektroden 6 und 7 abnimmt. Nach Applikation des Ausgleichstroms bestimmter Länge und Amplitude kann erneut die Spannung ermittelt werden. Wenn anschließend durch den Vergleicher 4 festgestellt wird, dass weiterhin eine elektrische Spannung zwischen den Elektroden 6, 7 außerhalb des vorgegebenen Spannungsbereichs vorliegt, so wird die Applikation eines weiteren Ausgleichstroms wiederholt.

Dieser Vorgang der alternierenden Ermittlung der elektrischen Spannung zwischen den Elektroden 6, 7 und der Applikation von Stromimpulsen zum Ausgleich von Unausgeglichenheiten elektrischer Ladungen an den Stimulationselektroden 6, 7 kann so oft wiederholt werden, bis die elektrische Spannung zwischen den Elektroden 6, 7 innerhalb des vorgegebenen Spannungsbereichs liegt bzw. die elektrische Ladung an den Stimulationselektroden 6, 7 ausgeglichen ist. Sobald die Spannung zwischen den Stimulationselektroden 6, 7 wieder innerhalb des vorgegebenen Spannungsbereichs liegt bzw. die elektrische Ladung an den Stimulationselektroden 6, 7 ausgeglichen ist, legt der Ladungsinjektor 3 keinen Strom mehr auf die Stimulationselektrode 6, indem beispielsweise die Stromerzeugung des Ladungsinjektors 3 abgeschaltet wird oder die Verbindung zwischen dem Ladungsinjektor 3 und der Stimulationselektrode 6 durch den Schaltkontakt 9 unterbrochen wird.

Wie bei der in Figur 1 dargestellten Ausführungsform der erfindungsgemäßen Ausführungsform ist es dabei ausreichend, dass der Ladungsinjektor 3 nur mit einer Stimulationselektrode 6 verbunden ist, da der Ladungsinjektor 3 in der Lage ist, eine positive oder eine negative Spannung mit der erforderlichen Stromstärke zu erzeugen, um einen Ladungsausgleich zwischen den Stimulationselektroden 6, 7 herbeizuführen. Ob eine positive oder eine negative Spannung und welche Stromstärke für den Ladungsausgleich zwischen den Stimulationselektroden 6, 7 erforderlich ist, wird zuvor durch den Ladungs- bzw. Spannungsmesser 5 und den Vergleicher 4 ermittelt und an den Ladungsinjektor 3 weitergeleitet. Durch diese aktiv gesteuerte Regulierung über den Ladungs- bzw. Spannungsmesser 5, den Vergleicher 4 und die Regelung des Ausgleichsstroms mittels des Ladungsinjektors 3 wird gewährleistet, dass die Spannung zwischen den Stimulationselektroden 6 und 7 bestimmte Grenzwerte nicht überschreitet bzw. die elektrische Spannung zwischen den Stimulationselektroden 6 und 7 abnimmt oder auf Null reduziert wird.

Die erfindungsgemäße Vorrichtung zum Ausgleich von Unausgeglichenheiten elektrischer Ladungen an den Stimulationselektroden kann allgemein in einem System zur Stimulation von Gewebe oder Nerven durch einzelne oder wiederholte Stimulationsimpulse über Stimulationselektroden angewendet werden und ist vorzugsweise in einem solchen Stimulations-System integriert. Gemäß einem besonderen Aspekt der vorliegenden Erfindung ist die erfindungsgemäße Vorrichtung zum Ausgleich von Unausgeglichenheiten elektrischer Ladungen an den Stimulationselektroden vorzugsweise nur dann aktiv, wenn über die Stromquelle 2 kein Laststrom bzw. kein Stimulationsstrom auf die Stimulationselektroden 6, 7 gelegt wird, d.h. wenn das mit den Stimulationselektroden (6, 7) kontaktierte Gewebe (8, 12) oder Nerven nicht durch Stimulationsimpulse der Stimulationselektroden (6, 7) stimuliert wird. Auf diese Weise können etwaige Unausgeglichenheiten elektrischer Ladungen an den Stimulationselektroden durch nahezu stromlose Messung besonders exakt ermittelt und ausgeglichen werden.

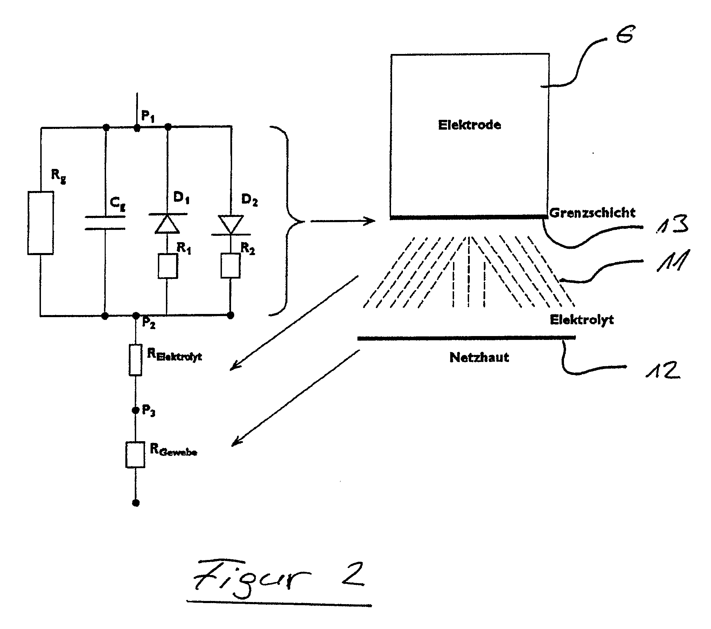

Figur 2 zeigt ein Elektrodenmodell in Form einer schematischen Darstellung mit einem elektrischen Ersatzschaltbild für eine Vorrichtung gemäß einer bevorzugten Ausführungsform der vorliegenden Erfindung zur Verwendung in einem Stimulations-System. In Figur 2 ist ein Modell zur Beschreibung der prinzipiellen Vorgänge an den Stimulationselektroden eines erfindungsgemäßen Stimulations-Systems dargestellt.

Das in Figur 2 dargestellte Elektrodenmodell der erfindungsgemäßen Vorrichtung wird anhand eines Retina-Stimulations-Systems zur Anwendung an einem menschlichen Auge als Beispiel erläutert, wobei von dem Retina-Stimulations-System lediglich eine Stimulationselektrode 6 dargestellt ist. Auf der rechten Seite der Figur 2 ist die Kontaktierung der Stimulationselektrode 6 des Retina-Stimulations-Systems mit der Netzhaut 12 eines menschlichen Auges schematisch dargestellt. Dabei steht die Stimulationselektrode 6 über einen Elektrolyt 11 mit der Netzhaut (Retina) 12 des menschlichen Auges in Kontakt, wobei zwischen der Stimulationselektrode 6 und dem Elektrolyt 11 eine Grenzschicht 13 ausgebildet ist. Der Elektrolyt 11 besteht im Wesentlichen aus einer wässrigen Lösung, in der sich elektrisch geladene Ionen befinden.

Auf der linken Seite der Figur 2 ist ein elektrisches Ersatzschaltbild zur Erläuterung der Funktionsweise der erfindungsgemäßen Vorrichtung dargestellt, wobei die Bezüge des jeweiligen Bauteils des Stimulations-Systems mit der betreffenden Komponente des Ersatzschaltbilds durch Pfeile angedeutet sind. Der in der Figur 2 von der geschweiften Klammer umfasste Teil des Ersatzschaltbildes dient der Darstellung der prinzipiellen Vorgänge an der Grenzschicht 13 zwischen der Stimulationselektrode 6 und dem Elektrolyt 11.

Das Ersatzschaltbild der Grenzschicht 13 zwischen der Stimulationselektrode 6 und dem Elektrolyt 11 umfasst einen ersten elektrischen Knotenpunkt P1, einen Grenzschichtwiderstand Rg, eine Kapazität Cg, eine erste Reihenschaltung bestehend aus einer ersten Diode D1 und einem ersten Widerstand R1, eine zweite Reihenschaltung bestehend aus einer zweiten Diode D2 und einem zweiten Widerstand R2 sowie einen zweiten elektrischen Knotenpunkt P2. Der Grenzschichtwiderstand Rg, die Kapazität Cg, die erste Reihenschaltung bestehend aus der ersten Diode D1 und der ersten Widerstand R1, die zweite Reihenschaltung bestehend aus der zweiten Diode D2 und dem zweiten Widerstand R2 sind jeweils zwischen dem ersten elektrischen Knotenpunkt P1 und dem zweiten elektrischen Knotenpunkt P2 parallel geschaltet.

Die erste Diode D1 ist innerhalb der ersten Reihenschaltung so angeschlossen, dass ihre Kathode mit dem elektrischen Knotenpunkt P1 verbunden ist, während die zweite Diode D2 innerhalb der zweiten Reihenschaltung so angeschlossen ist, dass ihre Anode mit dem elektrischen Knotenpunkt P1 verbunden ist. Zwischen dem zweiten elektrischen Knotenpunkt P2 und einem dritten elektrischen Knotenpunkt P3 ist ein Elektrolytwiderstand R_{Elektrolyt} angeschlossen, mit dem ein spezifischer Widerstand des Gewebes R_{Gewebe} verbunden ist.

Die einzelnen Komponenten des Ersatzschaltbildes haben in Bezug auf die Komponenten des Stimulations-Systems die folgenden physikalische Entsprechungen. Die Grenzschichtkapazität Cg entspricht der elektrischen Kapazität an der Grenzschicht 13 zwischen der Stimulationselektrode 6 und dem Elektrolyt 11. Die Grenzschichtkapazität Cg wird im Wesentlichen durch die Ausrichtung der im Elektrolyten 11 vorhandenen Wasser-Dipolmoleküle sowie durch die Anlagerung der im Elektrolyten 11 vorhandenen Ionen bestimmt. Während einer Stimulation der Netzhaut durch die Stimulationselektrode 6 werden bei einem ordnungsgemäßen Betrieb des Stimulations-Systems die im Elektrolyten vorhandenen Ionen nicht entladen. Die elektrische Kapazität an der Grenzschicht Cg wird ferner durch die wirksame Fläche der Elektrode 6 und durch die physikalischen Eigenschaften des Elektrolyten 11 bestimmt.

Der Grenzschichtwiderstand Rg beschreibt das Verhalten eines geringfügigen Ladungstransports innerhalb der Grenzschicht 13 zwischen dem Elektrolyt 11 und der Stimulationselektrode 6. Der Grenzschichtwiderstand Rg liegt im Bereich von 10 Megaohm. Innerhalb der Grenzschicht 13 können Ladungstransporte auftreten, ohne dass es zu nachteiligen Effekten kommen muss, die weiter unten beschrieben werden.

Der Elektrolytwiderstand R_{Elektrolyt} entspricht dem elektrischen Widerstand des Elektrolyts 11 und setzt sich im Wesenlichten durch die wirksame Oberfläche der Stimulationselektrode 6 und durch den spezifischen Widerstand des Elektrolyten 11 zusammen.

Der Widerstand der Netzhaut und der darunter liegenden subretinalen Gewebeschichten wird im Wesentlichen durch den spezifischen Widerstand des Gewebes R_{Gewebe} bestimmt. Dabei ist der spezifische Gewebewiderstand R_{Gewebe} größer, als der spezifische Widerstand R_{Elektrolyt} des Elektrolyten 11.

Zwischen den elektrischen Knotenpunkten P1 und P2 kann sich eine Spannung aufbauen, die im Folgenden mit U12 bezeichnet wird. Auch wenn die Spannung zwischen P1 und P2 die Durchbruchspannungen der Dioden D1 und D2 nicht überschreitet, kann innerhalb der Grenzschicht 13 ein Strom fließen, ohne dass dabei nachteilige Effekte auf die Stimulationselektrode 6 oder auf das Gewebe entstehen.

Die Wahl und die Anordnung der oben genannten elektrischen Komponenten des in Figur 2 angegebenen Ersatzschaltbildes ist möglichst einfach gehalten, um die prinzipiellen Vorgänge an der Grenzschicht 13 zwischen der Stimulationselektrode 6 und der Netzhaut 12 in übersichtlicher Weise darzustellen. Für eine exakte Abbildung der in der Realität zwischen der Stimulationselektrode 6 und der Netzhaut 12 auftretenden Effekte müssten weitere Komponenten zum Ersatzschaltbild hinzufügt werden. Insbesondere die Wahl der beiden gegenpolig angeordneten Dioden D1 und D2, ist daher lediglich symbolisch zu verstehen.

Nachfolgend werden unter Bezugnahme auf Figur 2 nachteilige Vorgänge beschrieben, die während des Betriebs eines Stimulations-Systems in der Stimulationselektrode 6, im Elektrolyt 11 zwischen der Stimulationselektrode 6 und der Netzhaut 12 sowie an der Grenzschicht 13 zwischen dem Elektrolyt 11 und der Stimulationselektrode 6 auftreten können. Die Dioden D1 und D2 sind dabei als ideale Elemente zu betrachten, deren Durchbruchspannungen vorzugsweise im Bereich von einigen Zehntel Volt liegen. Beim Betrieb der Stimulations-Systems können je nach anodischer oder katodischer Anregung der Dioden D1 und D2 durch das Überschreiten von diskreten Spannungen zwischen den elektrischen Knotenpunkten P1 und P2 über einen bestimmten Zeitraum verschiedene Effekte auftreten, die im Wesentlichen in vier Fallgruppen unterschieden werden können:
1. Die im Elektrolyt 11 enthaltenen Ionen gehen in Lösung und die Elektrode 6 löst sich auf.
2. Die im Elektrolyt 11 enthaltenen Ionen werden Entladen und die Elektrode 6 wächst.
3. Die Oberfläche der Elektrode 6 wird oxidiert oder reduziert.
4. Es entsteht eine Gasbildung im Elektrolyt 11.

Die oben genannten Effekte, die beim Betrieb des Stimulations-Systems in der Stimulationselektrode 6, im Elektrolyt 11 zwischen der Stimulationselektrode 6 und der Netzhaut 12 sowie an der Grenzschicht 13 zwischen dem Elektrolyt 11 und der Stimulationselektrode 6 auftreten können, sind für das stimulierte Gewebe und für eine einwandfreie Funktion des Stimulations-Systems von Nachteil. Es ist daher ein Ziel bei der Ansteuerung von Stimulationselektroden, die oben genannten Fallgruppen nachteiliger Effekte in jedem Fall zu vermieden. Dieses Ziel wird zum einen dadurch erreicht, dass die Spannung U12 den elektrischen Knotenpunkten P1 und P2 immer unter den Durchbruchspannungen der Dioden D1 und D2 gehalten wird. Um dies zu bewerkstelligen, müssen die verschiedenen Zustände der Stimulationselektroden betrachtet werden.

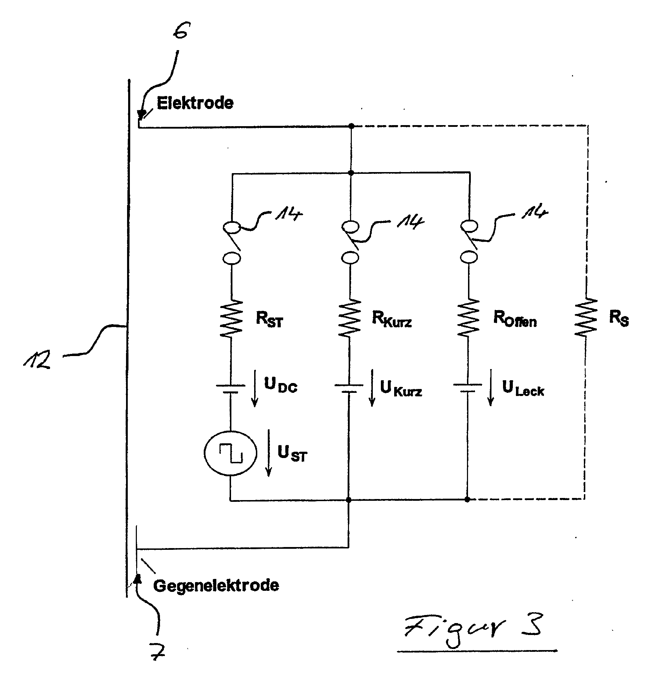

Figur 3 zeigt eine schematische Ersatzschaltung zur Darstellung unterschiedlicher Zustände der Stimulationselektroden eines Stimulations-Systems mit einer Vorrichtung gemäß einer bevorzugten Ausführungsform der vorliegenden Erfindung. Auch bei dieser Darstellung dient ein Retina-Stimulations-System als Beispiel für die Verwendung bzw. Funktionsweise der erfindungsgemäßen Vorrichtung, wobei von dem Stimulations-System in Figur 3 lediglich eine Stimulationselektrode 6 und eine Gegenelektrode 7 dargestellt ist. Gleichwohl umfasst ein Stimulations-System üblicherweise eine große Anzahl von Stimulationselektroden 6, 7, die jeweils mit der Netzhaut 12 eines menschlichen Auges kontaktiert sind.

Das in Figur 3 dargestellte Ersatzschaltbild umfasst mehrere parallel angeordnete Reihenschaltungen die jeweils einen Zustand der Stimulationselektroden 6, 7 wiedergeben, wobei für die Erläuterung der Elektrodenzustände auch auf das in Figur 2 dargestellte Ersatzschaltbild Bezug genommen wird. Jede der in Figur 3 dargestellten Reihenschaltung ist jeweils über ein Ende mit der Stimulationselektrode 6 und über das andere Ende mit der Stimulationselektrode 7 verbunden. Jede der Reihenschaltungen in Figur 3 weist jeweils einen Schalter 14 auf, über den die Verbindung der betreffenden Reihenschaltung zur Stimulationselektrode 6 unterbrochen bzw. hergestellt werden kann.

Die erste Reihenschaltung umfasst einen Widerstand R_{ST}, eine Spannungsquelle U_{DC} sowie eine Wechselstromquelle U_{ST}. Die zweite Reihenschaltung umfasst einen Widerstand R_{KURZ} und eine Spannungsquelle U_{KURZ}. Die dritte Reihenschaltung umfasst einen Widerstand R_{OFFEN} und eine Spannungsquelle U_{LECK}. Die vierte Reihenschaltung umfasst einen Widerstand R_{S}.

Bei einer Funktionsweise des Stimulations-Systems ohne Kurzschluss-Beschaltung der Stimulationselektroden 6, 7 kann eine Ansteuerungsfrequenz von etwa 60Hz zugrunde gelegt werden, so dass sich ein Stimulationszyklus für die Stimulationselektrode 6 von etwa 16,7 ms einstellt. Innerhalb dieses Stimulationszyklus wird die Stimulationselektrode über eine Zeitspanne von etwa 3 ms stimuliert. Das Anlegen einer elektrischen Spannung oder Ladung auf die Stimulationselektroden 6, 7 in einem Stimulationszyklus entspricht dabei dem Elektrodenzustand der ersten Reihenschaltung. Außerhalb des Stimulationszyklus ist die Stimulationselektrode 6 entweder kurzzeitig mit der Gegenelektrode 7 kurzgeschlossen, was dem Elektrodenzustand der zweiten Reihenschaltung entspricht, oder die Stimulationselektrode ist offen, d.h. nicht mit einer elektrischen Spannung oder Ladung beaufschlagt, was dem Elektrodenzustand der dritten Reihenschaltung entspricht.

Ausgehend von der Annahme, dass nur etwa 10 % aller Stimulationselektroden des Stimulations-Systems gleichzeitig angesteuert werden und diese Ansteuerung in etwa gleich verteilt ist, so wird eine Stimulationselektrode nur etwa 1,8 % der Betriebszeit des Stimulations-Systems mit einer elektrischen Spannung oder Ladung beaufschlagt. Auch bei einer Nicht-Gleichverteilung der Ansteuerung der Stimulationselektroden überwiegt in jedem Fall die Zeit mit ca. 90% der Betriebszeit des Stimulations-Systems, in der sich die Elektrode im Leerlauf befindet bzw. offen ist, d.h. nicht mit einer elektrischen Spannung oder Ladung beaufschlagt wird.

Während dieser Zeit kann sich die in Figur 2 dargestellte Kapazität Cg über den Leckstrom I_{leck} aufladen, der über die Spannungsquelle U_{LECK} fließt. Dabei wird davon ausgegangen, dass der in Figur 2 dargestellte Widerstand Rg etwa 10 MOhm beträgt und die Spannung U12 zwischen den in Figur 2 dargestellten elektrischen Knotenpunkten etwa 200mV nicht überschreiten darf. Um eine der oben genannten nachteiligen Effekte zu vermeiden, muss I_{leck}< 20 nA sein. Wird der Kondensator Cg zwischenzeitlich entladen, kann der Leckstrom I_{leck} entsprechend größer sein. Wäre der Widerstand Rg dagegen unendlich groß, müsste I_{leck} gleich 0 sein, um einen ordnungsgemäßen Betrieb der erfindungsgemäßen Vorrichtung zu gewährleisten.

Bei einer Stimulation wird von einem Stimulationsimpulsstrom von maximal 1 mA ausgegangen. Ein Abweichung von 0,01% entspricht dann einem Gleichstrom von 100 nA. Wenn die Elektrode maximal 10% der Zeit stimuliert wird, so resultiert dies in einem Gleichstrom von 10 nA. Das bedeutet, dass ohne einen Ladungsausgleich zwischen den Stimulationsimpulsen ein Charge-Balance bzw. ein Ladungsausgleich zwischen den Stimulationselektroden etwa in derselben Größenordnung erfolgen muss.

Eine Möglichkeit zur direkten Messung oder Überwachung der Spannung U12 zwischen dem ersten elektrischen Knotenpunkt P1 und dem zweiten elektrischen Knotenpunkt P2 ist nicht gegeben, da bei einer Messung der Gesamtspannung U12 auch die Spannungsabfälle über dem Elektrolyten 11 und dem Netzhaut-Gewebe 12 gemessen werden. Eine Spannungs- oder Restpotentialmessung zwischen dem ersten elektrischen Knotenpunkt P1 und dem zweiten elektrischen Knotenpunkt P2 ist daher zunächst nur zu solchen Zeitpunkten möglich, wenn gerade keine Stimulation an den Stimulationselektroden 6,7 stattfindet, also quasi stromlos gemessen wird.

Wie dem in Figur 3 dargestellten Ersatzschaltbild zu entnehmen ist, kann optional ein Schutzwiderstand R_{S} eingefügt werden, der einerseits mit der Elektrode 6 und andererseits mit der Gegenelektrode 7 verbunden ist; die Anschlüsse des Schutzwiderstands R_{S} sind mit gestrichelten Verbindungslinien dargestellt. Dieser Schutzwiderstand R_{S} kann etwa im Bereich von 100 kOhm liegen und würde bei einer Charge-Imbalance bzw. einer Unausgeglichenheit der elektrische Ladung zwischen den Stimulationselektroden 6, 7 von etwa 1 % ausreichen um den Kondensator Cg zwischen den Stimulationsphasen zu entladen.

Zusätzlich oder alternativ besteht auch die Möglichkeit, der Kondensator Cg zwischen den Stimulationselektroden 6, 7 durch Kurzschließen der Elektrode 6 mit der Gegenelektrode 7 zu entladen. Dies könnte beispielsweise zwischen zwei Stimulationsimpulsen durch einen Kurzschluss der Elektrode 6 mit der Gegenelektrode 7 für ca. 3 ms erfolgen. Dabei ist jedoch zu beachten, dass in dieser Zeit der Entladung durch Kurzschließen einer Elektrode ihrer Gegenelektrode keine Nachbarelektroden stimuliert werden, die sich in der Nähe der kurzgeschlossenen Stimulationselektroden befinden.

Die vorliegende Erfindung wurde lediglich durch ein Anwendungsbeispiel anhand des Elektrodenmodells und des Ersatzschaltbildes im Zusammenhang mit einem Retina-Stimulations-System zur Anwendung an einem menschlichen Auge erläutert. Selbstverständlich können die durch die Patentansprüche definierte erfindungsgemäße Vorrichtung sowie das erfindungsgemäße Verfahren auch in anderen Stimulations-Systemen angewendet werden.

### Liste der Bezugszeichen

- 1: Impulsgenerator
- 2: Strom/Spannungsquelle
- 3: Ladungsinjektor
- 4: Vergleicher
- 5: Ladungs- bzw. Spannungsmesser
- 6: Stimulationselektrode
- 7: Stimulationselektrode bzw. Gegenelektrode
- 8: lebendes Gewebe oder Nerven
- 9: Schaltkontakt
- 10: Schaltkontakt
- 11: Elektrolyt
- 12: Netzhaut (Retina)
- 13: Grenzschicht zwischen Stimulationselektrode 6 und Elektrolyt 11
- 14: Schaltkontakt

## Patentansprüche

1. Vorrichtung zur Stimulation von lebendem Gewebezellen oder Nerven durch einzelne oder wiederholte Stimulationsimpulse über Stimulationselektroden (6, 7), wobei die Stimulationselektroden (6, 7) mit lebenden Nervenzellen oder Gewebe (8, 12) kontaktiert sind, das durch die Stimulationsimpulse der Stimulationselektroden (6, 7) stimuliert wird,
**dadurch gekennzeichnet,**
**dass** die Vorrichtung eine elektrische Schaltung (1, 2, 3, 4, 5) umfasst, welche die elektrische Spannung bzw. die elektrische Ladung an den Stimulationselektroden (6, 7) in Abhängigkeit von der elektrischen Spannung zwischen den Stimulationselektroden (6, 7) regelt um Unausgeglichenheiten elektrischer Ladungen an den Stimulationselektroden (6, 7) zu reduzieren oder auszugleichen.

2. Vorrichtung nach Anspruch 1, wobei die Vorrichtung, einen positiven oder negativen elektrischen Strom bestimmter Dauer und Intensität erzeugt und an mindestens eine Stimulationselektrode (6) anlegt, wodurch Unausgeglichenheiten elektrischer Ladungen zwischen den Stimulationselektroden (6, 7) reduziert oder ausgeglichen werden.

3. Vorrichtung nach einem der Ansprüche 1 oder 2, wobei die Vorrichtung einen Impulsgenerator (1) umfasst, der elektrische Impulse erzeugt, die von einer Strom/Spannungsquelle (2) zu Stimulationsimpulsen verstärkt und zumindest an eine erste Stimulationselektrode (6), vorzugsweise an eine Anzahl von Stimulationselektroden (6, 7) weitergeleitet werden.

4. Vorrichtung nach einem der vorangehenden Ansprüche, wobei die Form oder der Verlauf der von der Vorrichtung erzeugten elektrischen Stimulationsimpulse oder die Stromimpulse für den Ladungsausgleich zwischen den Stimulationselektroden (6, 7) auf die Art des zu stimulierenden Gewebes (8, 12) oder der zu stimulierenden Nervenzellen angepasst ist.

5. Vorrichtung nach einem der vorangehenden Ansprüche, wobei die Verbindung der elektrischen (1, 2, 3, 4, 5) Schaltung zu der mindestens einen Stimulationselektrode 6 durch einen Schaltkontakt 10 unterbrochen oder hergestellt werden kann.

6. Vorrichtung nach einem der vorangehenden Ansprüche, wobei die Vorrichtung einen Ladungs- und/oder Spannungsmesser (5) umfasst, der mit den Stimulationselektroden (6, 7) verbunden ist und die elektrische Spannung zwischen den Stimulationselektroden (6, 7) oder Unausgeglichenheiten an den Stimulationselektroden (6, 7) ermittelt.

7. Vorrichtung nach Anspruch 6, wobei der Ladungs- und/oder Spannungsmesser (5) die elektrische Spannung zwischen den Stimulationselektroden (6, 7) zwischen Stimulationszyklen ermittelt, d.h. während das mit den Stimulationselektroden (6, 7) kontaktierte Gewebe (8, 12) oder Nerven nicht durch Stimulationsimpulse der Stimulationselektroden (6, 7) stimuliert wird oder kein Laststrom oder Stimulationsstrom an den Stimulationselektroden (6, 7) anliegt.

8. Vorrichtung nach einem der vorangehenden Ansprüche, wobei die Vorrichtung einen Vergleicher (4) umfasst, der feststellt, ob die elektrische Spannung oder die Ladungsverschiedenheiten zwischen den Stimulationselektroden (6, 7) unterhalb, innerhalb oder oberhalb eines bestimmten Spannungsbereichs liegt, der durch vorgegebene Grenzwerte definiert ist.

9. Vorrichtung nach einem der vorangehenden Ansprüche, wobei die Vorrichtung einen Ladungsinjektor (3) umfasst, der einen positiven oder negativen elektrischen Strom bestimmter Dauer und Intensität erzeugt und an die Stimulationselektrode (6) anlegt, wodurch Unausgeglichenheiten elektrischer Ladungen an den Stimulationselektroden (6, 7) reduziert oder ausgeglichen werden.

10. Vorrichtung nach einem der Ansprüche 6 bis 9, wobei das durch den Ladungs- und/oder Spannungsmesser (5) ermittelte Resultat bezüglich der elektrischen Spannung zwischen den Stimulationselektroden (6, 7) oder der Unausgeglichenheiten elektrischer Ladungen an den Stimulationselektroden (6, 7) an den Vergleicher (4) weitergeleitet wird und das Ergebnis des vom Vergleicher (4) durchgeführten Vergleichs, ob die elektrische Spannung oder die Ladungsverschiedenheiten zwischen den Stimulationselektroden (6, 7) unterhalb, innerhalb oder oberhalb eines vorgegebenen Spannungsbereichs liegt, an den Ladungsinjektor (3) weitergeleitet wird und der Ladungsinjektor (3) aufgrund der vom Vergleicher (4) weitergeleiteten Signale eine positive oder eine negative Spannung und mit bestimmter Stromstärke über eine bestimmte Zeitspanne an die mindestens eine Stimulationselektrode (6) anlegt, so dass die elektrische Spannung zwischen den Stimulationselektroden (6, 7) oder Unausgeglichenheiten elektrischer Ladungen an den Stimulationselektroden (6, 7) reduziert oder ausgeglichen werden.

11. Vorrichtung gemäß einem der vorangehenden Ansprüche, wobei die Vorrichtung zur Elektrostimulation einer Retina eines Auges, insbesondere in Abhängigkeit von einfallendem Licht dient, mit einer elektrischen Schaltung (1, 2, 3, 4, 5) vorzugsweise in Form eines integrierten Schaltkreis, der dazu ausgebildet ist, im Bereich der Retina implantiert zu werden, wobei die elektrische Schaltung (1, 2, 3, 4, 5) eine Anzahl von Kontaktstellen zum Kontaktieren von Retinazellen und eine Anzahl von lichtempfindlichen Elementen beinhaltet, die in Abhängigkeit von auftreffendem Licht die Kontaktstellen über die elektrische Schaltung (1, 2, 3, 4, 5) ansteuern.

12. Vorrichtung nach einem der vorangehenden Ansprüche, wobei die elektronischen Komponenten (1, 2, 3, 4, 5) der elektrischen Schaltung sowie metallische Leiterbahnen zu deren Kontaktierung photolithographisch mikrostrukturiert und vorzugsweise auf einem Chip untergebracht sind.

13. System zur Stimulation von lebendem Gewebe (8, 12) oder Nerven durch einzelne oder wiederholte Stimulationsimpulse über Stimulationselektroden (6, 7) mit einer Vorrichtung gemäß einem der vorangehenden Ansprüche, wobei die Vorrichtung vorzugsweise in dem Stimulations-System integriert ist.

14. Verfahren zum Betreiben der Vorrichtung nach einem der vorangehenden Ansprüche umfassend die folgenden Schritte:
• Ermitteln einer elektrischen Spannung zwischen den Stimulationselektroden (6, 7) oder einer Unausgeglichenheit elektrischer Ladungen zwischen den Stimulationselektroden (6, 7),
• Vergleichen der ermittelten Spannung zwischen den Stimulationselektroden (6, 7) mit einem vorgegebenen Spannungsbereich,
**gekennzeichnet durch**
• Erzeugen und Anlegen eines positiven oder negativen elektrischen Stroms bestimmter Dauer und Intensität an mindestens eine Stimulationselektrode (6), wodurch die elektrischen Spannung zwischen den Stimulationselektroden (6, 7) oder eine Unausgeglichenheit elektrischer Ladungen an den Stimulationselektroden (6, 7) reduziert oder ausgeglichen wird.

15. Verfahren nach Anspruch 14, wobei die elektrische Spannung zwischen den Stimulationselektroden (6, 7) auch während des Ladungsausgleichs an den Stimulationselektroden (6, 7) ermittelt wird und sobald die Spannung zwischen den Stimulationselektroden (6, 7) wieder innerhalb des vorgegebenen Spannungsbereichs liegt oder keine Spannung mehr zwischen den Stimulationselektroden (6, 7) vorliegt, die Stromzufuhr zum Ladungsausgleich zu den Stimulationselektroden (6, 7) unterbrochen wird.

16. Verfahren nach einem der Ansprüche 14 oder 15, wobei die Ermittlung einer elektrischen Spannung zwischen den Stimulationselektroden (6, 7) oder einer Unausgeglichenheit elektrischer Ladungen an den Stimulationselektroden (6, 7) und/oder der Ladungsausgleich an den Stimulationselektroden (6, 7) zwischen Stimulationszyklen durchgeführt wird, d.h. während das mit den Stimulationselektroden (6, 7) kontaktierte Gewebe (8, 12) oder Nerven nicht durch Stimulationsimpulse der Stimulationselektroden (6, 7) stimuliert wird oder kein Laststrom oder Stimulationsstrom an den Stimulationselektroden (6, 7) anliegt.

17. Verfahren nach einem der Ansprüche 14 bis 16, wobei die Ermittlung einer elektrischen Spannung zwischen den Stimulationselektroden (6, 7) oder einer Unausgeglichenheit elektrischer Ladungen an den Stimulationselektroden (6, 7) nur dann durchgeführt wird, wenn kein Stimulationsstrom an die Stimulationselektroden (6, 7) angelegt ist.

18. Verfahren nach einem der Ansprüche 13 bis 17, wobei die Ermittlung einer elektrischen Spannung zwischen den Stimulationselektroden (6, 7) oder einer Unausgeglichenheit elektrischer Ladungen an den Stimulationselektroden (6, 7) und/oder der Ladungsausgleich an den Stimulationselektroden (6, 7) nur dann durchgeführt wird, wenn an den Stimulationselektroden (6, 7) und an Stimulationselektroden in der Nähe der Stimulationselektroden (6, 7) kein Stimulationsstrom angelegt ist, d.h. weder die Stimulationselektroden (6, 7) noch Stimulationselektroden in der Nähe der Stimulationselektroden (6, 7) durch Stimulationsimpulse stimuliert werden.

19. Verfahren nach einem der Ansprüche 14 bis 18, wobei die Ermittlung einer elektrischen Spannung zwischen den Stimulationselektroden (6, 7) oder einer Unausgeglichenheit elektrischer Ladungen an den Stimulationselektroden (6, 7) und/oder der Ladungsausgleich an den Stimulationselektroden (6, 7) zyklisch wiederholt wird.

20. Verfahren nach einem der Ansprüche 14 bis 19, wobei die Ermittlung einer elektrischen Spannung zwischen den Stimulationselektroden (6, 7) oder einer Unausgeglichenheit elektrischer Ladungen an den Stimulationselektroden (6, 7) und die Zufuhr eines Ausgleichsstroms mit bestimmter Dauer und Amplitude zu den Stimulationselektroden (6, 7) zum Ladungsausgleich an den Stimulationselektroden (6, 7) alternierend durchgeführt wird.

21. System zur Stimulation von lebendem Gewebe (8, 12) oder Nerven durch einzelne oder wiederholte Stimulationsimpulse über Stimulationselektroden (6, 7), das nach einem Verfahren gemäß einem der Ansprüche 13 bis 18 arbeitet.

## Claims

1. A device for stimulating living tissue cells or nerves by individual or repeated stimulating pulses via stimulating electrodes (6, 7), the stimulating electrodes (6, 7) being contacted with living nerve cells or tissue (8, 12) being stimulated by the stimulating pulses of the stimulating electrodes (6, 7),
**characterized in that**
the device comprises an electrical circuit (1, 2, 3, 4, 5) which controls the electric voltage or the electric charge on the stimulating electrodes (6, 7) as a function of the electric voltage between the stimulating electrodes (6, 7) for reducing or equalising imbalances of electric charges on the stimulating electrodes (6, 7).

2. Device according to Claim 1, wherein the device generates a positive or negative electric current of defined duration and intensity and applies it to at least one stimulating electrode (6), as a result of which imbalances of electric charges between the stimulating electrodes (6, 7) are reduced or equalised.

3. Device according to one of Claims 1 or 2, wherein the device includes a pulse-generator (1) which generates electrical pulses which are amplified into stimulating pulses by a current/voltage source (2) and passed to at least one first stimulating electrode (6), preferably to a number of stimulating electrodes (6, 7).

4. Device according to one of the preceding claims, wherein the shape or contour of the electrical stimulating pulses generated by the device or of the current pulses for the charge equalisation between the stimulating electrodes (6, 7) is adapted to the type of tissue (8, 12) to be stimulated or to the type of nerve cells to be stimulated.

5. Device according to one of the preceding claims, wherein the connection of the electrical circuit (1, 2, 3, 4, 5) to the at least one stimulating electrode 6 can be interrupted or established by a switching contact 10.

6. Device according to one of the preceding claims, wherein the device includes a coulombmeter and/or voltmeter (5) which is connected to the stimulating electrodes (6, 7) and ascertains the electric voltage between the stimulating electrodes (6, 7) or ascertains imbalances at the stimulating electrodes (6, 7).

7. Device according to Claim 6, wherein the coulombmeter and/or voltmeter (5) ascertains the electric voltage between the stimulating electrodes (6, 7) between stimulation cycles, i.e. while the tissue (8, 12) or nerves contacted with the stimulating electrodes (6, 7) is/are not being stimulated by stimulating pulses of the stimulating electrodes (6, 7), or no load current or stimulating current is being applied to the stimulating electrodes (6, 7).

8. Device according to one of the preceding claims, wherein the device includes a comparator (4) which establishes whether the electric voltage or the differences in charge between the stimulating electrodes (6, 7) lies below, within or above a defined voltage range which is defined by predetermined limiting values.

9. Device according to one of the preceding claims, wherein the device includes a charge-injector (3) which generates a positive or negative electric current of defined duration and intensity and applies it to the stimulating electrode (6), as a result of which imbalances of electric charges on the stimulating electrodes (6, 7) are reduced or equalised.

10. Device according to one of Claims 6 to 9, wherein the result ascertained by the coulombmeter and/or voltmeter (5) with respect to the electric voltage between the stimulating electrodes (6, 7) or with respect to the imbalances of electric charges on the stimulating electrodes (6, 7) is passed to the comparator (4) and the result of the comparison carried out by the comparator (4) as to whether the electric voltage or the differences in charge between the stimulating electrodes (6, 7) lies below, within or above a predetermined voltage range is passed to the charge-injector (3) and on the basis of the signals passed by the comparator (4) the charge-injector (3) applies a positive or a negative voltage and with defined current intensity over a defined time-interval to the at least one stimulating electrode (6), so that the electric voltage between the stimulating electrodes (6, 7) or imbalances of electric charges on the stimulating electrodes (6, 7) are reduced or equalised.

11. Device according to one of the preceding claims, wherein the device serves for electrostimulation of a retina of an eye, in particular as a function of incident light, with an electrical circuit (1, 2, 3, 4, 5) preferably in the form of an integrated circuit which is designed to be implanted in the region of the retina, wherein the electrical circuit (1, 2, 3, 4, 5) includes a number of contact-points for contacting retinal cells and a number of light-sensitive elements which drive the contact-points via the electrical circuit (1, 2, 3, 4, 5) as a function of incident light.

12. Device according to one of the preceding claims, wherein the electronic components (1, 2, 3, 4, 5) of the electrical circuit and also metallic conductor tracks for the contacting thereof are photolithographically microstructured and preferably accommodated on a chip.

13. A system for stimulating living tissue (8, 12) or nerves by individual or repeated stimulating pulses via stimulating electrodes (6, 7), with a device according to one of the preceding claims, wherein the device is preferably integrated within the stimulation system.

14. A process for operating the device according to one of the preceding claims, comprising the following steps:
• ascertaining an electric voltage between the stimulating electrodes (6, 7) or ascertaining an imbalance of electric charges between the stimulating electrodes (6, 7),
• comparing the ascertained voltage between the stimulating electrodes (6, 7) with a predetermined voltage range,
**characterized by**
• generating and applying a positive or negative electric current of defined duration and intensity to at least one stimulating electrode (6), as a result of which the electric voltage between the stimulating electrodes (6, 7) or an imbalance of electric charges on the stimulating electrodes (6, 7) is reduced or equalised.

15. Process according to Claim 14, wherein the electric voltage between the stimulating electrodes (6, 7) is ascertained also during the equalisation of charge on the stimulating electrodes (6, 7) and as soon as the voltage between the stimulating electrodes (6, 7) again lies within the predetermined voltage range or no voltage is present any longer between the stimulating electrodes (6, 7) the supply of current to the stimulating electrodes (6, 7) for the purpose of charge equalisation is interrupted.

16. Process according to one of Claims 14 or 15, wherein the ascertainment of an electric voltage between the stimulating electrodes (6, 7) or of an imbalance of electric charges on the stimulating electrodes (6, 7) and/or of the equalisation of charge on the stimulating electrodes (6, 7) is carried out between stimulation cycles, i.e. while the tissue (8, 12) or nerves contacted with the stimulating electrodes (6, 7) is/are not being stimulated by stimulating pulses of the stimulating electrodes (6, 7) or no load current or stimulating current is being applied to the stimulating electrodes (6, 7).

17. Process according to one of Claims 14 to 16, wherein the ascertainment of an electric voltage between the stimulating electrodes (6, 7) or of an imbalance of electric charges on the stimulating electrodes (6, 7) is only carried out when no stimulating current is applied to the stimulating electrodes (6, 7).

18. Process according to one of Claims 13 to 17, wherein the ascertainment of an electric voltage between the stimulating electrodes (6, 7) or of an imbalance of electric charges on the stimulating electrodes (6, 7) and/or of the equalisation of charge on the stimulating electrodes (6, 7) is only carried out when no stimulating current is applied to the stimulating electrodes (6, 7) and to stimulating electrodes in the vicinity of the stimulating electrodes (6, 7), i.e. neither the stimulating electrodes (6, 7) nor stimulating electrodes in the vicinity of the stimulating electrodes (6, 7) are being stimulated by stimulating pulses.

19. Process according to one of Claims 14 to 18, wherein the ascertainment of an electric voltage between the stimulating electrodes (6, 7) or of an imbalance of electric charges on the stimulating electrodes (6, 7) and/or of the equalisation of charge on the stimulating electrodes (6, 7) is repeated cyclically.

20. Process according to one of Claims 14 to 19, wherein the ascertainment of an electric voltage between the stimulating electrodes (6, 7) or of an imbalance of electric charges on the stimulating electrodes (6, 7) and the supply of an equalising current with defined duration and amplitude to the stimulating electrodes (6, 7) for the purpose of equalisation of charge on the stimulating electrodes (6, 7) is carried out in alternating manner.

21. A system for stimulating living tissue (8, 12) or nerves by individual or repeated stimulating pulses via stimulating electrodes (6, 7), said system operating by a process according to one of Claims 13 to 18.

## Revendications

1. Dispositif de stimulation de cellules tissulaires vivantes ou de nerfs par des impulsions de stimulation uniques ou répétées par le biais d'électrodes de stimulation (6, 7), les électrodes de stimulation (6, 7) étant mises en contact avec des cellules nerveuses vivantes ou un tissu (8, 12) qui est stimulé par les impulsions de stimulation des électrodes de stimulation (6, 7),
**caractérisé en ce que**
le dispositif comprend un circuit électrique (1, 2, 3, 4, 5) qui régule la tension électrique ou respectivement la charge électrique au niveau des électrodes de stimulation (6, 7) en fonction de la tension électrique entre les électrodes de stimulation (6, 7) afin de réduire ou d'équilibrer des déséquilibres de charges électriques au niveau des électrodes de stimulation (6, 7).

2. Dispositif selon la revendication 1, le dispositif produisant un courant électrique positif ou négatif d'une durée et d'une intensité déterminées et l'appliquant à au moins une électrode de stimulation (6), ce qui réduit ou équilibre des déséquilibres de charges électriques entre les électrodes de stimulation (6, 7).

3. Dispositif selon l'une des revendications 1 ou 2, le dispositif comprenant un générateur d'impulsions (1) qui produit des impulsions électriques qui sont amplifiées par une source de courant/tension (2) en impulsions de stimulation et conduites au moins à une première électrode de stimulation (6), de préférence à un certain nombre d'électrodes de stimulation (6, 7).

4. Dispositif selon l'une des revendications précédentes, dans lequel la forme ou l'allure des impulsions de stimulation électriques produites par le dispositif ou les impulsions de courant pour l'équilibrage des charges entre les électrodes de stimulation (6, 7) est adaptée(s) au type du tissu (8, 12) à stimuler ou des cellules nerveuses à stimuler.

5. Dispositif selon l'une des revendications précédentes, dans lequel la connexion du circuit électrique (1, 2, 3, 4, 5) à l'électrode de stimulation (6) au moins au nombre de un peut être interrompue ou établie par un contact de commutation (10).

6. Dispositif selon l'une des revendications précédentes, le dispositif comprenant un appareil de mesure de charge et/ou de tension (5) qui est connecté aux électrodes de stimulation (6, 7) et qui détermine la tension électrique entre les électrodes de stimulation (6, 7) ou des déséquilibres au niveau des électrodes de stimulation (6, 7).

7. Dispositif selon la revendication 6, dans lequel l'appareil de mesure de charge et/ou de tension (5) détermine la tension électrique entre les électrodes de stimulation (6, 7) entre des cycles de stimulation, c'est-à-dire pendant que le tissu (8, 12) ou des nerfs mis en contact avec les électrodes de stimulation (6, 7) n'est pas/ne sont pas stimulé(s) par des impulsions de stimulation des électrodes de stimulation (6, 7) ou pendant qu'aucun courant de charge ou aucun courant de stimulation n'est présent au niveau des électrodes de stimulation (6, 7).

8. Dispositif selon l'une des revendications précédentes, le dispositif comprenant un comparateur (4) qui constate si la tension électrique ou les disparités de charges entre les électrodes de stimulation (6, 7) est/sont située(s) au-dessous, à l'intérieur ou au-dessus d'une plage de tension déterminée qui est définie par des valeurs limites prédéterminées.

9. Dispositif selon l'une des revendications précédentes, le dispositif comprenant un injecteur de charge (3) qui produit un courant électrique positif ou négatif de durée et d'intensité définies et l'applique à l'électrode de stimulation (6), ce qui réduit ou équilibre des déséquilibres de charges électriques au niveau des électrodes de stimulation (6, 7).

10. Dispositif selon l'une des revendications 6 à 9, dans lequelle résultat déterminé par l'appareil de mesure de charge et/ou de tension (5) concernant la tension électrique entre les électrodes de stimulation (6, 7) ou les déséquilibres de charges électriques au niveau des électrodes de stimulation (6, 7) est transmis au comparateur (4), et le résultat de la comparaison effectuée par le comparateur (4) et indiquant si la tension électrique ou les disparités de charges entre les électrodes de stimulation (6, 7) est/sont située(s) au-dessous, à l'intérieur ou au-dessus d'une plage de tension prédéterminée est transmis à l'injecteur de charge (3), et l'injecteur de charge (3) applique, sur la base des signaux transmis par le comparateur (4), une tension positive ou une tension négative, et avec une intensité de courant définie sur une période de temps définie, à l'électrode de stimulation (6) au moins au nombre de un de telle sorte que la tension électrique entre les électrodes de stimulation (6, 7) ou des déséquilibres de charges électriques au niveau des électrodes de stimulation (6, 7) est/sont réduite(s) ou équilibrée(s).

11. Dispositif conformément à l'une des revendications précédentes, le dispositif servant à l'électrostimulation d'une rétine d'un oeil, en particulier en fonction de la lumière incidente, avec un circuit électrique (1, 2, 3, 4, 5) de préférence sous la forme d'un circuit intégré qui est constitué pour être implanté dans la zone de la rétine, le circuit électrique (1, 2, 3, 4, 5) contenant un certain nombre d'emplacements de contact pour la mise en contact avec des cellules de la rétine et un certain nombre d'éléments photosensibles qui, en fonction de la lumière incidente, pilotent les emplacements de contact par le biais du circuit électrique (1, 2, 3, 4, 5).

12. Dispositif selon l'une des revendications précédentes, dans lequel les composants électriques (1, 2, 3, 4, 5) du circuit électrique ainsi que des pistes de conduction métalliques pour leur mise en contact sont microstructurés par photolithographie et de préférence mis en place sur une puce.

13. Système de stimulation de tissus vivants (8, 12) ou de nerfs par des impulsions de stimulation uniques ou répétées par le biais d'électrodes de stimulation (6, 7) avec un dispositif conforme à une des revendications précédentes, le dispositif étant de préférence intégré dans le système de stimulation.

14. Procédé de fonctionnement du dispositif selon l'une des revendications précédentes, comprenant les étapes suivantes :
• détermination d'une tension électrique entre les électrodes de stimultion (6, 7) ou d'un déséquilibre de charges électriques entre les électrodes de stimulation (6, 7),
• comparaison de la tension déterminée entre les électrodes de stimulation (6, 7) avec une plage de tension prédéterminée,
**caractérisé par**
• la production et l'application d'un courant électrique positif ou négatif d'une durée et d'une intensité déterminées au niveau d'au moins une électrode de stimulation (6), ce qui réduit ou équilibre la tension électrique entre les électrodes de stimulation (6, 7) ou un déséquilibre de charges électriques au niveau des électrodes de stimulation (6, 7).

15. Procédé selon la revendication 14, dans lequel la tension électrique entre les électrodes de stimulation (6, 7) est déterminée également pendant l'équilibrage de charges au niveau des électrodes de stimulation (6, 7), et l'amenée de courant aux électrodes de stimulation (6, 7) pour l'équilibrage de charges est interrompue dès que la tension entre les électrodes de stimulation (6, 7) est de nouveau à l'intérieur de la plage de tension prédéterminée ou dès qu'il n'y a plus de tension entre les électrodes de stimulation (6, 7).

16. Procédé selon l'une des revendications 14 ou 15, dans lequel la détermination d'une tension électrique entre les électrodes de stimulation (6, 7) ou d'un déséquilibre de charges électriques au niveau des électrodes de stimulation (6, 7) et/ou l'équilibrage de charges au niveau des électrodes de stimulation (6, 7) sont effectués entre des cycles de stimulation, c'est-à-dire pendant que le tissu (8, 12) ou des nerfs mis en contact avec les électrodes de stimulation (6, 7) n'est pas/ne sont pas stimulé(s) par des impulsions de stimulation des électrodes de stimulation (6, 7) ou qu'aucun courant de charge ou courant de stimulation n'est présent au niveau des électrodes de stimulation (6, 7).

17. Procédé selon l'une des revendications 14 à 16, dans lequel la détermination d'une tension électrique entre les électrodes de stimulation (6, 7) ou d'un déséquilibre de charges électriques au niveau des électrodes de stimulation (6, 7) n'est effectuée que si aucun courant de stimulation n'est appliqué aux électrodes de stimulation (6, 7).

18. Procédé selon l'une des revendications 13 à 17, dans lequel la détermination d'une tension électrique entre les électrodes de stimulation (6, 7) ou d'un déséquilibre de charges électriques au niveau des électrodes de stimulation (6, 7) et/ou l'équilibrage de charges au niveau des électrodes de stimulation (6, 7) ne sont effectués que si aucun courant de stimulation n'est appliqué au niveau des électrodes de stimulation (6, 7) et au niveau d'électrodes de stimulation à proximité des électrodes de stimulation (6, 7), c'est-à-dire si ni les électrodes de stimulation (6, 7) ni des électrodes de stimulation à proximité des électrodes de stimulation (6, 7) ne sont stimulées par des impulsions de stimulation.

19. Procédé selon l'une des revendications 14 à 18, dans lequel la détermination d'une tension électrique entre les électrodes de stimulation (6, 7) ou d'un déséquilibre de charges électriques au niveau des électrodes de stimulation (6, 7) et/ou l'équilibrage de charges au niveau des électrodes de stimulation (6, 7) sont répétés de façon cyclique.

20. Procédé selon l'une des revendications 14 à 19, dans lequel la détermination d'une tension électrique entre les électrodes de stimulation (6, 7) ou d'un déséquilibre de charges électriques au niveau des électrodes de stimulation (6, 7) et l'amenée d'un courant d'équilibrage d'une durée et d'une amplitude déterminées aux électrodes de stimulation (6, 7) pour l'équilibrage de charges au niveau des électrodes de stimulation (6, 7) sont effectuées de façon alternée.

21. Système de stimulation de tissus vivants (8, 12) ou de nerfs par des impulsions de stimulation uniques ou répétées par le biais d'électrodes de stimulation, (6, 7) qui fonctionne selon un procédé conforme à l'une des revendications 13 à 18.
